Europäisches Patentamt

(19) European Patent Office

Office européen des brevets

(11) Publication number: **0 102 733**
**B1**

(12) **EUROPEAN PATENT SPECIFICATION**

(45) Date of publication of patent specification: **10.12.86**

(21) Application number: **83304305.2**

(22) Date of filing: **26.07.83**

(51) Int. Cl.⁴: **A 61 B 5/02,** A 61 B 10/00,
A 61 B 8/06

(54) **Ultrasonic pulse Doppler blood flow meter.**

(30) Priority: **28.07.82 JP 131602/82**

(43) Date of publication of application:
**14.03.84 Bulletin 84/11**

(45) Publication of the grant of the patent:
**10.12.86 Bulletin 86/50**

(84) Designated Contracting States:
**DE FR GB NL SE**

(56) References cited:
**EP-A-0 027 215**
**AT-B- 351 146**
**US-A-4 060 763**
**US-A-4 122 713**
**US-A-4 143 650**

(73) Proprietor: **FUJITSU LIMITED**
**1015, Kamikodanaka Nakahara-ku**
**Kawasaki-shi Kanagawa 211 (JP)**

(72) Inventor: **Amemiya, Shin-ichi**
**c/o FUJITSU LIMITED 1015 Kamikodanaka**
**Nakahara-ku Kawasaki (JP)**

(74) Representative: **Sunderland, James Harry et al**
**HASELTINE LAKE & CO Hazlitt House 28**
**Southampton Buildings Chancery Lane**
**London WC2A 1AT (GB)**

## Description

This invention relates to an ultrasonic pulse Doppler blood flow meter.

An ultrasonic pulse Doppler blood flow meter measures blood flow rate and its distribution by transmitting ultrasonic wave pulses into living body tissue and receiving waves reflected from blood corpuscles. Such meters have suffered disadvantages in that many manual adjustments are required for their operation and on the whole they are not easy-to-operate items of equipment.

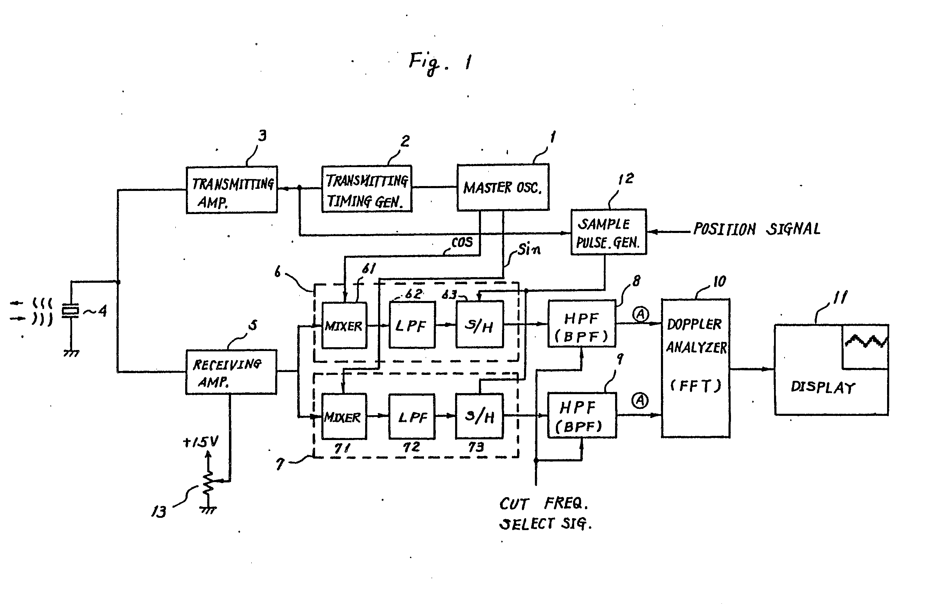

Figure 1 is a schematic block diagram of a previously proposed ultrasonic pulse Doppler blood flow meter.

In Figure 1, 1 is a master oscillator; 2 is a transmission timing generator which generates a transmission timing signal by division of an oscillation output of the master oscillator; 3 is a transmission amplifier which generates a pulse or burst transmission signal; 4 is a transducer (ultrasonic wave probe) which generates an ultrasonic wave signal, for transmission into a living body in accordance with the transmission signal and receives the wave signal as reflected from the living body; and 5 is a receiving amplifier which amplifies a reflected wave signal received by the transducer.

13 is a variable resistor manually adjustable for controlling the gain of receiving amplifier 5. 6 and 7 are real (R) and imaginary (I) Doppler element detectors.

The detectors 6 and 7 have respective mixers 61 and 71 which receive cos and sin signals, differing in phase by 90°, from the master oscillator and carry out orthogonal detection, respective low-pass-filters (LPF) 62 and 72 and respective sample hold circuits (S/H) 63 and 73.

The detector 6 detects real elements of a Doppler signal reflected from a specified depth (depth: distance between the probe and the location generating a reflected wave), while the detector 7 detects imaginary elements of the Doppler signal.

The meter of Figure 1 further comprises high-pass-filters (HPF) 8 and 9 which are provided to eliminate low frequency Doppler elements from the outputs of the detectors 6 and 7. Such low frequency elements are generated by the wall of the heart of the living body. HPF cut-off frequency can be set on the basis of a cut frequency select signal. 10 is a Doppler analyzer which is provided with an AD converter and digital processor and which carries out frequency analysis in respect of the outputs of both filters 8 and 9 by a fast Fourier transformation (FFT). The outputs of the filters are Doppler elements indicating blood flow rate. 11 is a display for indicating the results of such analysis. 12 is a sampling pulse generator which generates sampling pulses for the sample hold circuits 63 and 73 on the basis of a position designation signal and an output of the transmission timing generator 2.

As explained above, since cos and sin reference signals, differing in phase by 90°, are supplied to the mixers 61 and 71 from the master oscillator, orthogonal detection is carried out by the detectors 6 and 7. Gain of receiving amplifier 5 can be adjusted by a variable resistor 13 for gain control which is provided at an operation panel of the meter.

In the Doppler blood flow meter of Figure 1, since the high-pass-filters (HPF's) 8 and 9 have an upper limit on their input levels (about ±10V), HPF output level is low when the Doppler signal contains a low frequency element due to movement of the heart wall and therefore the Doppler analyzer 10 must have high accuracy, in order to be able to deal with such low output levels. Otherwise, a satisfactory analysis cannot be effected. However, other problems remain:—
(1) the brightness of the display 11 must be adjusted so as to be suitable to the HPF output level; (2) cut-off frequency of the HPF's 8 and 9 must be changed in accordance with blood flow rate; (3) gain of the receiving amplifier must also be changed in accordance with the level of received signals.

Attention is directed to US—A—4 143 650.

According to the present invention there is provided an ultrasonic pulse Doppler blood flow meter, comprising:

an ultrasonic transducer operable to receive ultrasonic waves reflected from living body tissue,

a receiving amplifier operable to amplify a signal provided by the transducer in response to received ultrasonic waves,

a Doppler detector operable to mix the amplified signal output of the receiving amplifier with a reference signal to generate a Doppler signal therefrom;

a high pass filter operable to cut low frequency elements from the Doppler signal;

a Doppler analyzer operable to carry out a Doppler analysis on the basis of the output of the high pass filter, and

a display for displaying the result of analysis by the analyzer, and characterised by

an amplitude equalizing circuit connected between the high pass filter and the Doppler analyzer, operable at least approximately to equalize the amplitude of the output of the high pass filter, and to pass on the so equalized output of the high pass filter to the Doppler analyzer for Doppler analysis.

An embodiment of the present invention can mitigate the problems posed by the need for manual adjustment of amplifier gain and manual selection of filter cut-off frequency in the previously proposed meter, and can enable circuit stages following a receiving amplifier to perform efficiently so as efficiently to use the analyzing accuracy of a Doppler analyzer, by employing an AGC type receiving amplifier 5 and by providing an amplitude equalizing circuit after an HPF stage of an ultrasonic pulse blood flow meter.

An embodiment of the present invention achieves characteristic control of a receiving circuit of an ultrasonic blood flow meter.

In an embodiment of this invention, the receiving amplifier may be an automatic gain control type receiving amplifier operable to provide an output amplified signal of substantially constant amplitude from a probe signal derived from ultrasonic waves reflected at the specified depth.

Reference is made, by way of example, to the accompanying drawings, in which:—

Figure 1 is a schematic block diagram of a previously proposed ultrasonic pulse Doppler blood flow meter,

Figure 2 is a schematic outline block diagram of an amplitude equalizing circuit in accordance with an embodiment of this invention,

Figure 3 is a more detailed diagram of the circuit of Figure 2,

Figure 4 is a graph for assistance in explaining advantages which can be obtained by the use of an amplitude equalizing circuit as shown in Figure 2,

Figure 5 is a circuit diagram of a receiving amplifier, of an automatic gain control type, as employed in an embodiment of the present invention, and

Figure 6 is a schematic circuit diagram of a gate circuit which can be used in place of sample hold circuits as employed in the Doppler detector shown in Figure 1, and

Figure 7 illustrates an alternative form of a part of the circuit of Figure 3 which allows the amplification factor of a multiplier thereof to be selectively altered.

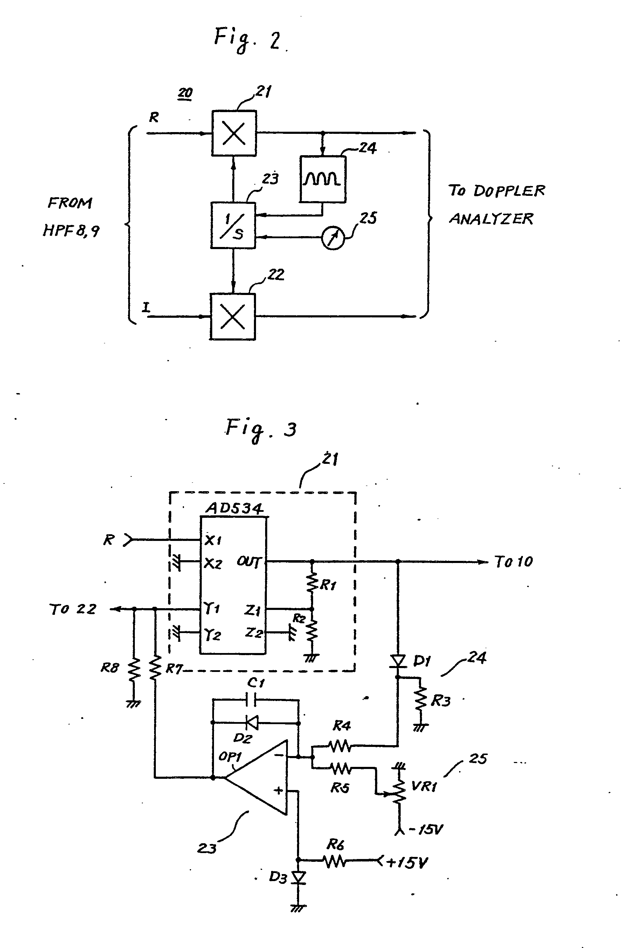

Figure 2 illustrates in outline a part of an embodiment of this invention; that is, it illustrates an amplitude equalizing circuit 20 which in an embodiment of this invention is provided in a meter as shown in Figure 1, at the points Ⓐ—Ⓐ in Figure 1.

In Figure 2, 21 is a multiplier which receives as one input an output R of HPF 8. 22 is a multiplier which receives as one input an output I of HPF 9. The multipliers 21 and 22 receive further inputs from an integrating circuit 23 which set the multiplication factors of the multipliers in accordance with the output amplitude of HPF 8.

24 is a full-wave or half-wave detector for detecting the output amplitude of HFP 8 (after multiplication in multiplier 21). 25 is a level setter which designates a desired signal level.

In operation, any difference between the outputs of detector 24 and level setter 25 is integrated by the integrating circuit 23. The result is delivered as a gain (multiplication factor) control signal to the multiplier 21 which is a variable gain amplifier. A similar control signal is provided for multiplier 22. Thereby, an average value $\sqrt{R^2+I^2}$ of the signals R and I to be applied to the Doppler analyzer 10 is set constant (for example, to 5Vp—p).

In the embodiment of the present invention illustrated by reference to Figure 2, only the output of the multiplier 21 is detected for the purpose of controlling both of the multipliers 21 and 22, because the amplitudes of the signals R and I are in practice almost equal. Alternatively, of course, the outputs of both multipliers 21 and 22 (and hence the outputs of both HPF's 8 and 9) may be detected.

Figure 3 is a diagram of a practical circuit structure for the circuit of Figure 2. Multiplier 21 comprises an integrated circuit AD 534 and resistors $R_1$, $R_2$. The multiplier 22 has the same structure but for clarity is omitted from Figure 3. A diode $D_1$ and resistor $R_3$ provide half-wave detector 24. Resistors $R_4$, $R_5$ and a level setting variable resistor $VR_1$ provide an adder circuit and a difference provided by the adder (difference between detector and level setter outputs) is input to the integrating circuit 23. The integrating circuit 23 comprises an operational amplifier $OP_1$ and other elements i.e. $R_6R_8$, diodes $D_2$, $D_3$ and capacitor $C_1$. Of these other elements, feedback gain and level adjustment response rate are determined by a time constant $C_1R_4$. An adequate time constant is about a single Doppler analysis time (about 30 msec). The diode $D_2$ prevents the output of the operational amplifier $OP_1$ becoming negative. The diode $D_3$ is connected to the positive (reference) side input of the operational amplifier OP in order to compensate for a voltage drop of about 0.7V across the diode $D_2$. The resistors $R_7$, $R_8$ divide the maximum output value of operational amplifier OP (about 12V when the power source of +15V is used) to a value suitable for input to the multiplier 21. For example, when an input limit of AD 534 is 10V, the maximum output value of OP is divided into 10/12 by the resistors $R_6$, $R_8$. The resistors $R_1$, $R_2$ of multiplier 21 are used for setting a maximum amplification factor, which is expressed by the following equation when the integrated circuit AD 534 is used.

Maximum amplification degree

$$=(R_1+R_2)/R_2.$$

Two important advantages are obtained by providing the amplitude equalizing circuit 20 at points Ⓐ—Ⓐ, in the meter of Figure 1.

First, since input amplitude to the Doppler analyzer is fixed by the circuit 20, even if gain control up to HPF 8 and 9 is inadequate, the dynamic range of an A/D converter involved in the input stage of the analyzer or of a digital processor in successive stages of the analyzer can be effectively utilized. Thereby, adjustment of brightness of the display 11 is no longer necessary.

Second, since filter cut-off frequency changes automatically, frequent manual adjustment of a cut-frequency selection signal (see Figure 1) is no longer necessary.

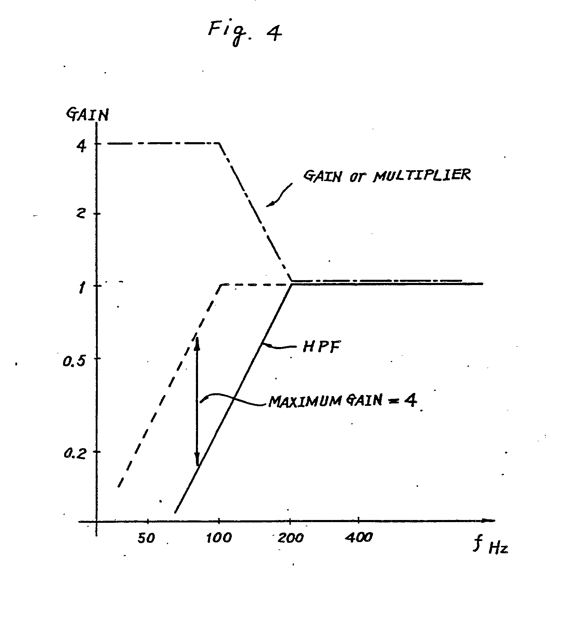

Figure 4 is a graph illustrating frequency characteristic (gain v. frequency) for assistance in explaining this latter advantage.

When the transfer characteristic of HPF 8 or 9 for a+10V input signal is as indicated in Figure 4 by a solid line, the transfer characteristic of the meter including amplitude equalizing circuit 20 can be as indicated by a broken line in Figure 4.

Namely, assuming the HPF transfer characteristic is as indicated by the solid line in Figure 4, when an input signal to HPF has a frequency as low as 200 Hz or less, HPF output level drops. Accordingly, in the embodiment of the present invention, the gain of the amplitude equalizing circuit 20 increases as indicated by the chain line in Figure 4, becoming constant at a maximum value at very low frequencies. Therefore, the overall transfer characteristic is extended into a lower frequency region as indicated by the broken line in Figure 4.

Above an input signal of a single frequency has been considered.

When high frequency components generated by blood flow and low frequency components generated by heart wall movement coexist in an input signal, the frequency characteristics are as follows.

When blood flow rate is high and Doppler frequency is 400 Hz, for example, the output level of HPF is high and the amplitude equalizing circuit does not operate to increase that amplitude and therefore the overall characteristic is as shown by the solid line in Figure 4 and a frequency component of about 100 Hz generated by heart wall movement is effectively suppressed.

On the other hand, when blood flowrate is low, corresponding to a frequency of about 100 Hz, heart wall movement is also low and the Doppler frequency in respect of that movement is as low as 30 Hz. At any rate, since input signal frequencies are lower than the HPF cut-off, HPF output level is low and the amplitude equalizing circuit comes into operation and accordingly an overall characteristic as shown by the broken line in Figure 4 can be obtained. Namely, a sufficient gain is obtained in respect of the signal frequency relating to blood flow (100 Hz) but substantially no gain is obtained in respect of the signal frequency relating to heart wall movement (30 Hz). In this case also, the blood flow component and heart wall component, namely signal and noise, can be isolated.

Thus, with the circuit of Figure 2, the filter cut-off frequency is automatically reduced when blood flow rate is low, and automatically raised when blood flow rate is high, and thereby unwanted low frequency components arising from heart wall movement etc. can be cut.

If, on the other hand, the HPF frequency characteristic were not modified as in the embodiment of the present invention with the circuit of Figure 2, with a low blood flow rate only noise would be obtained. In the previously proposed meter this can be avoided only by manual adjustment of the cut-frequency fc of HPF. However, according to the above-described embodiment of this invention, such adjustment is executed automatically and since amplitude is adjusted for this purpose, the dynamic range of stages of the meter succeeding the HPF's, namely the Doppler analyzer, can be efficiently utilized.

When no distortion is generated in the meter circuits preceding the Doppler detectors 6 and 7, a high precision Doppler analysis can be carried out as a result of the effects of the amplitude equalizing circuit 20 as explained above.

Since the output level of the transducer or probe of the flow meter, in response to received reflected ultrasonic signals, can vary over a wide range, gain control of the receiving amplifier 5 is necessary in order to eliminate such distortion. If gain has to be adjusted manually, as it is in the case of the meter of Figure 1, the operations involved are very complicated and the effects obtained are not adequate for the purpose.

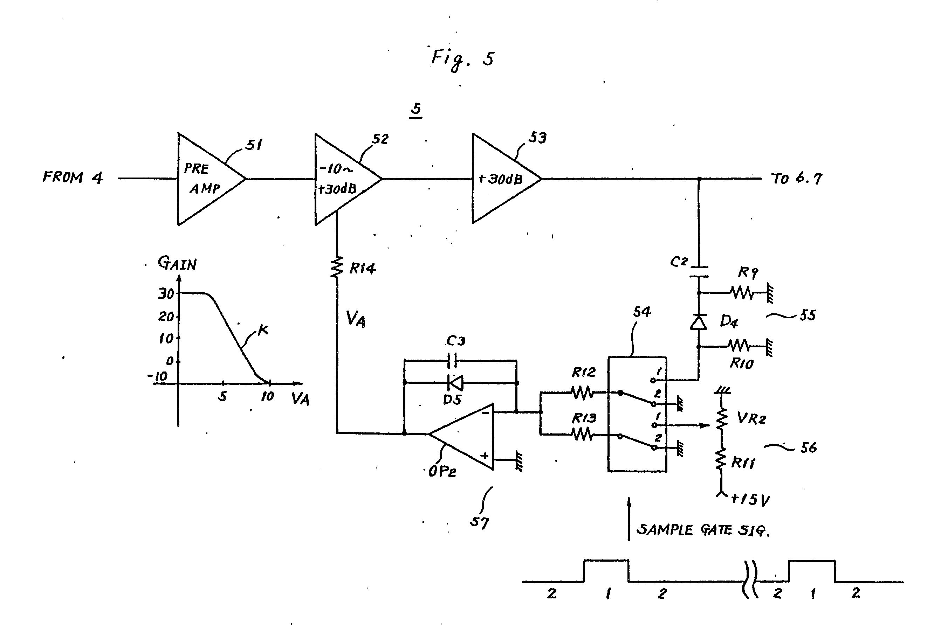

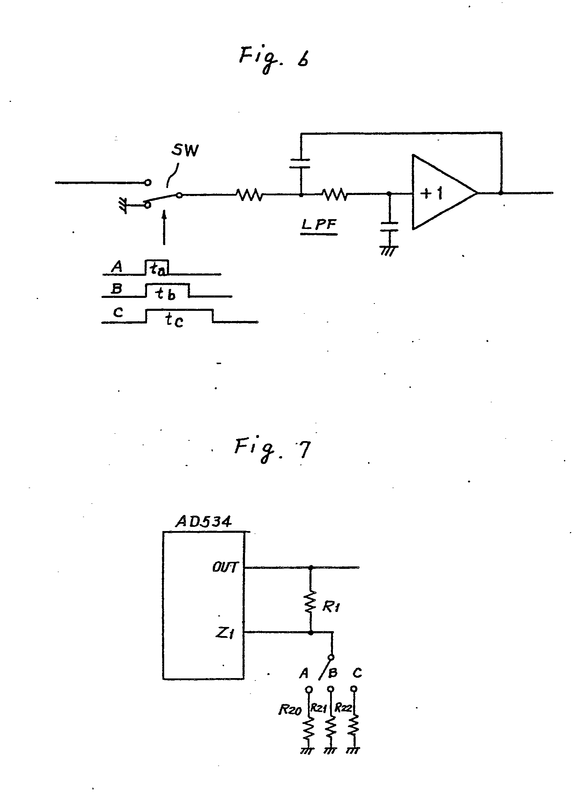

Figure 5 is a circuit diagram of a receiving amplifier 5 as employed in an embodiment of this invention for avoiding such problems.

The receiving amplifier 5 of Figure 5 has, connected in series, a preamplifier 51, which amplifies a received signal, an AGC amplifier 52 of $-10\sim+30$ dB, and an amplifier 53 with a gain of $+30$ dB. The feed back path of the AGC amplifier 52 is provided with a half-wave rectification detector comprising a diode $D_4$ and a resistor $R_9$, a level setter 56 comprising the variable resistor $VR_2$ and fixed resistor $R_{11}$, and an integrating circuit 57 comprising an operational amplifier $OP_2$, a diode $D_5$, a capacitor $C_3$ and resistors $R_{12}$, $R_{13}$, $R_{14}$.

An ordinary AGC amplifier drops the gain as the gain control voltage $V_A$ increases, as shown by the curve K in the graph inset in Figure 5. Therefore, unlike Figure 3, the diode $D_4$ is connected in reverse polarity and level setting is carried out for a positive voltage.

54 is an analog switch which provides a signal level corresponding to a sample position only, by means of a sample gate signal. Only when the switch 54 is set to terminals 1 (a sample position) is feedback provided for the AGC amplifier 52. Both inputs of operational amplifier $OP_2$ are grounded when the switch 54 is set to terminals 2 and the gain of amplifier 52 is thereby fixed. The level setting is also performed through the analog switch in order to prevent any change of output level due to the sample gate width. The AGC amplifier 52 is inserted at an intermediate position (between the preamplifier 51 and amplifier 53) because, (1) it is difficult to use the AGC amplifier in a first position because of high level noise in signals from the probe or transducer 4, and (2) maximum amplitude changes since DC bias changes. In order to make the maximum amplitude constant amplifier 53 is inserted after AGC amplifier 52.

In an embodiment of this invention a gate circuit as illustrated by Figure 6 can be used in place of the sample hold circuits 63 and 73 of the Doppler detectors 6 and 7 as shown in Figure 1. This gate circuit comprises a switch SW and a low-pass-filter LPF as shown in Figure 6 and can provide for changes in sample volume (size) by changes in the widths ta, tb, . . . of gate signals A, B . . . applied to the switch. When gate signal width is changed as described above, output amplitude changes, but such changes can be absorbed by the amplitude equalizing circuit 20 shown in Figure 2.

Further, if the gain of integrated circuit AD 534 used in multipliers 21, 22 is changed by means of the gate signals A, B, C, as shown in Figure 7, the maximum amplification factor (gain) also changes and a more effective result can be obtained. The resistors $R_{20}$ to $R_{22}$ correspond to the resistor $R_2$ of Figure 3 and have the following relationship to one another:

$$R_{20} < R_{21} < R_{22}.$$

In this case, the following relationship subsists between the gate widths ta, tb, . . . and the gains

$$ta[(R_1+R_{20})/R_{20}] \leqslant tb[(R_1+R_{21})/R_{21}] \leqslant tc[(R_1+R_{22})/R_{22}]$$

As described above, an embodiment of this invention can provide that manual gain adjustment and cut-off frequency adjustment are no longer necessary, improving operationability. In addition, the amplitude of the input to a Doppler analyzer is always maintained at a sufficiently high level and accuracy is much improved. Of course, in an embodiment of this invention, provision to permit manual selection of cut-off frequency of a high-pass-filter can be made. Moreover, since the range over which HPF cut-off frequency can be adjusted automatically is about fc/2, the range over which fc can be changed can be greatly increased in combination with the manual adjustment.

An embodiment of this invention provides an ultrasonic pulse Doppler blood flow meter in which an amplitude equalizing circuit is provided between a high-pass filter, which follows a Doppler detector, and a Doppler analyzer. This amplitude equalizing circuit not only enables the input dynamic range of the Doppler analyzer to be effectively utilized but also automatically adjusts the cut-frequency of the high-pass-filter in accordance with the condition of input signal. Therefore, a blood flow meter which requires few manual adjustments can be provided.

Although the embodiments of the present invention described above utilize a single transducer for ultrasonic wave transmission and reception, it will be understood that separate transmission and reception transducers may be employed.

## Claims

1. An ultrasonic pulse Doppler blood flow meter, comprising:
an ultrasonic transducer (4) operable to receive ultrasonic waves reflected from living body tissue,
a receiving amplifier (5) operable to amplify a signal provided by the transducer (4) in response to received ultrasonic waves,
a Doppler detector (6, 7) operable to mix the amplified signal output of the receiving amplifier (5) with a reference signal to generate a Doppler signal therefrom,
a high pass filter (8, 9) operable to cut low frequency elements from the Doppler signal,
a Doppler analyzer (10) operable to carry out a Doppler analysis on the basis of the output of the high pass filter (8, 9), and
a display (11) for displaying the result of analysis by the analyzer, and
characterised by
an amplitude equalizing circuit (20) connected between the high pass filter (8, 9) and the Doppler analyzer (10), operable at least approximately to equalize the amplitude of the output of the high pass filter, and to pass on the so equalized output of the high pass filter to the Doppler analyzer (10) for Doppler analysis.

2. A meter as claimed in claim 1, wherein the amplitude equalizing circuit comprises a multiplier (21) operable to amplitude-multiply the output of the high pass filter (8, 9), a detector (24) operable to detect the level of the amplitude-multiplied output, a level setter (25) operable to designate a desired level for the amplitude-multiplied output, and an integrator (23) operable to integrate differences between the detected level of the amplitude-multiplied output and the desired level, the multiplication factor of the multiplier (21) being set in dependence upon the integration result provided by the integrator (23).

3. A meter as claimed in claim 1 or 2, wherein the receiving amplifier (5) is of the automatic gain control type operable to provide the said amplified signal at a level which is at least approximately constant.

4. A meter as claimed in claim 3, wherein the receiving amplifier (5) comprises a preamplifier (51), an AGC amplifier (52), and a fixed gain amplifier (53) connected in series.

5. A meter as claimed in any preceding claim, wherein the Doppler detector (6) comprises a gate circuit, constituted by a switch (SW) and a low pass filter (LPF).

## Patentansprüche

1. Ultraschallimpuls - Doppler - Blutflußmesser mit:
einem Ultraschallwandler (4), der Ultraschallwellen empfangen kann, die von lebendem Körpergewebe reflektiert werden,
einem Empfangsverstärker (5), der ein Signal, welches von dem Wandler (4) geliefert wird, in Abhängigkeit von empfangenen Ultraschallwellen verstärken kann,
einem Doppler-Detektor (6, 7), der das verstärkte Ausgangssignal des Empfangsverstärkers (5) mit einem Referenzsignal mischen kann, um daraus ein Doppler-Signal zu bilden,
einem Hochpaßfilter (8, 9), der untere Frequenzelemente von dem Dopplersignal abschneiden kann,
einem Doppleranalysator (10), der eine Doppler-Analyse auf der Basis des Ausgangs des Hochpaßfilters (8, 9) durchführen kann, und

einer Anzeigevorrichtung (11) zum Anzeigen des Ergebnisses der Analyse durch den Analysator,

und gekennzeichnet durch

eine Amplitudenausgleichsschaltung (20), die zwischen dem Hochpaßfilter (8, 9) und dem Doppler-Analysator (10) angeschlossen ist und betreibbar ist, um wenigstens angenähert die Amplitude des Ausgangs des Hochpaßfilters auszugleichen, und um den so ausgeglichenen Ausgang des Hochpaßfilters zu dem Doppler-Analysator (10) für die Doppler-Analyse weiterzugeben.

2. Messer nach Anspruch 1, bei welchem die Amplitudenausgleichsschaltung einen Vervielfacher (21), der den Ausgang des Hochpaßfilters (8, 9) amplitudenvervielfachen kann, einen Detektor (24), der den Pegel des amplitudenvervielfachten Ausgangs detektieren kann, einen Pegeleinsteller (25), der einen gewünschten Pegel für den amplitudenvervielfachten Ausgang bestimmen kann, und einen Integrator (23), der Unterschiede zwischen dem detektierten Pegel des amplitudenvervielfachten Ausgangs und des gewünschten Pegels integrieren kann, umfaßt, wobei der Multiplikationsfaktor des Vervielfachers (21) in Abhängigkeit von dem Integrationsergebnis eingestellt wird, welches von dem Integrator (23) geliefert wird.

3. Messer nach Anspruch 1 oder 2, bei welchem der Empfangsverstärker (5) vom automatischen Verstärkungsreglertyp betreibbar ist, um das genannte verstärkte Signal mit einem Pegel zu liefern, welcher wenigstens angenähert konstant ist.

4. Messer nach Anspruch 3, bei welchem der Empfangsverstärker (5) einen Vorverstärker (51), einen AGC-Verstärker (52) und einen Verstärker (53) mit fester Verstärkung umfaßt, welche in Reihe verbunden sind.

5. Messer nach einem der vorhergehenden Ansprüche, bei welchem der Doppler-Detektor (6) eine Torschaltung umfaßt, die aus einem Schalter (SW) und einem Tiefpaßfilter (LPF) besteht.


**Revendications**

1. Débitmètre sanguin Doppler à impulsions ultrasonores, comprenant:

un transducteur d'ultrasons (4) ayant pour fonction de recevoir des ondes ultrasonores réfléchies par le tissu d'un être vivant,

um amplificateur de réception (5) ayant pour fonction d'amplifier un signal produit par le transducteur (4) en réponse à la réception d'ondes ultrasonores,

un détecteur Doppler (6, 7) ayant pour fonction de mélanger le signal de sortie amplifié de l'amplificateur de réception (5) avec un signal de référence afin de produire un signal Doppler,

un filtre passe-haut (8, 9) ayant pour fonction de couper les éléments de basse fréquence du signal Doppler,

un analyseur Doppler (10) ayant pour fonction d'effectuer une analyse Doppler sur la base du signal de sortie du filtre passe-haut (8, 9), et

un dispositif d'affichage (11) servant à afficher le résultat de l'analyse faite par l'analyseur,

et caractérisé par:

un circuit d'égalisation d'amplitude (20) connecté entre le filtre passe-haut (8, 9) et l'analyseur Doppler (10), ayant pour fonction d'égaliser au moins approximativement l'amplitude du signal de sortie du filtre passe-haut et de laisser passer le signal de sortie ainsi égalisé du filtre passe-haut à destination de l'analyseur Doppler (10) en vue de l'analyse Doppler.

2. Débitmètre selon la revendication 1, où le circuit d'égalisation d'amplitude comprend un multiplicateur (21) ayant pour fonction de multiplier l'amplitude du signal de sortie du filtre passe-haut (8, 9), un détecteur (24) ayant pour fonction de détecter le niveau du signal de sortie à amplitude multipliée, un moyen de fixation de niveau (25) ayant pour fonction d'indiquer un niveau voulu pour le signal de sortie à amplitude multipliée, et un intégrateur (23) ayant pour fonction d'intégrer les différences existant entre le niveau détecté du signal de sortie à amplitude multipliée et le niveau voulu, le facteur de multiplication du multiplicateur (21) étant fixé en fonction du résultat de l'intégration exécutée par l'intégrateur (23).

3. Débitmètre selon la revendication 1 ou 2, où l'amplificateur de réception (5) est du type à commande automatique de gain et a pour fonction de produire ledit signal amplifié à un niveau qui est au moins approximativement constant.

4. Débitmètre selon la revendication 3, où l'amplificateur de réception (5) comprend un préamplificateur (51), un amplificateur à commande automatique de gain (52), et un amplificateur à gain fixe (53) connectés en série.

5. Débitmètre selon l'une quelconque des revendications précédentes, où le détecteur Doppler (6) comprend un circuit de porte constitué par un commutateur (SW) et un filtre passe-bas (LPF).

Fig. 1

*Fig. 2*

FROM
HPF 8,9

To DOPPLER
ANALYZER

*Fig. 3*

AD534

Fig. 4

*Fig. 5*

5

FROM 4 → PRE AMP (51) → −10~+30dB (52) → +30dB (53) → To 6.7

R14, $V_A$

OP2 (57), C3, D5

R12, R13, 54, R9 (55), D4, R10, C2

VR2, R11, +15V (56)

SAMPLE GATE SIG.

GAIN graph: 30, 20, 10, 0, −10 vs $V_A$ (5, 10), curve K

Fig. 6

Fig. 7